# EUROPEAN PATENT APPLICATION

(11) **EP 3 831 409 A1**
(43) Date of publication of application: **09.06.2021**
(21) Application number: 19844718.7
(22) Date of filing: 31.07.2019
(51) Int. Cl.: A61K 45/00, A61K 31/711, A61K 45/06, A61P 19/02, A61P 19/08, A61P 19/10, A61P 29/00, A61P 35/00, A61P 37/02, A61P 43/00, C12N 9/99, C12N 15/113, C12Q 1/48, C12Q 1/6809, G01N 33/15, G01N 33/50

(54) **THERAPEUTIC AGENT FOR CARTILAGE DISEASE**

(30) Priority: 01.08.2018 JP 2018145324
(71) Applicant: ONO Pharmaceutical Co., Ltd., Osaka-shi, Osaka 541-8526 (JP)
(72) Inventor: TSUMORI, Yuki, Mishima-gun, Osaka 618-8585 (JP); NAKANISHI, Yasutomo, Mishima-gun, Osaka 618-8585 (JP); FUJIMURA, Shinsei, Mishima-gun, Osaka 618-8585 (JP)
(74) Representative: Grünecker Patent- und Rechtsanwälte PartG mbB
(86) International application number: PCT/JP2019/030022
(87) International publication number: WO 2020/027200

(57) **Abstract**

The present disclosure addresses the problem of providing a novel therapeutic agent, for cartilage disease, that has cartilage-regenerating action. Because substances having SIK2 inhibitory action have chondrocyte-differentiation-inducing action, a therapeutic agent formed so as to contain a substance having SIK2-inhibitory action is useful as a therapeutic agent, having cartilage-regenerating action, for cartilage damage or cartilage diseases such as osteoarthritis.

### [Sequence Listing]

SEQUENCE LISTING

<110>  ONO Pharmaceutical. CO., LTD

<120>  Therapeutic agent for cartilage related disease

<130>  674976

<150>  JP 2018-145324
<151>  2018-08-01

<160>  6

<170>  PatentIn version 3.5

<210>  1
<211>  16
<212>  DNA
<213>  Artificial Sequence

<220>
<223>  SIK2 antisense oligonucleotide

<400>  1
acaacaactg cacttt                                                       16


<210>  2
<211>  16
<212>  DNA
<213>  Artificial Sequence

<220>
<223>  SIK2 antisense oligonucleotide

<400>  2
aaccggccat gattag                                                       16


<210>  3
<211>  16
<212>  DNA
<213>  Artificial Sequence

<220>
<223>  SIK2 antisense oligonucleotide

<400>  3
cgatggatgg ctcatt                                                       16


<210>  4
<211>  16
<212>  DNA
<213>  Artificial Sequence

<220>
<223>  SIK2 antisense oligonucleotide

<400>  4
gacaataatc aacagc                                                       16

<210>  5
<211>  16
<212>  DNA
<213>  Artificial Sequence

<220>
<223>  SIK2 antisense oligonucleotide

<400>  5
tcaacagcag acagga                                                        16


<210>  6
<211>  16
<212>  DNA
<213>  Artificial Sequence

<220>
<223>  SIK3 antisense oligonucleotide

<400>  6
ggttagtgcc acgttg                                                        16

## Description

### TECHNICAL FIELD

This patent application claims priority to Japanese Patent Application No. 2018-145324, which is incorporated herein by reference in its entirety.

The present disclosure relates to a therapeutic agent for a cartilage-related disease (hereinafter, may be referred to as the therapeutic agent of the present disclosure), containing a substance having a salt-inducible kinase 2 (hereinafter, SIK2) inhibitory action.

### BACKGROUND ART

Articular cartilage is composed of hyaline cartilage lacking vascular, nerve and lymph, and thus is considered as a tissue that has very poor regeneration ability. Various factors such as genetic factor, injury and the like promote release of proteoglycan from the articular cartilage and at the same time, collagen type 2 which is fibrotic protein specifically existing in the hyaline cartilage is decomposed. By these consecutive reaction, degeneration or destruction of articular cartilage tissue progresses irreversibly. As a result, an articulation loses smooth mobility, and disorders such as inability to absorb an external impact occur, which leads to cartilage disease. In critical condition, an articulation loses the function itself. In addition, aging is considered as one of the causes of cartilage disease and the number of patients is expected to increase in a future aging society.

According to these circumstances, radical treatment of cartilage-related diseases is eagerly desired, but most of current treatment methods are mainly symptomatic treatments such as inflammation suppression and pain suppression with non-steroidal anti-inflammatory agents. Injection of hyaluronic acid preparation does not also lead to cartilage tissue regeneration. In recent years, microfracture method, autologous osteochondral transplantation (mosaicplasty), and autologous chondrocyte implantation have been performed as new therapies for the purpose of cartilage regeneration. However, they are inferior in versatility because treatment target is limited to traumatic cartilage defect or osteochondritis dissecans and size of each target affected site is limited. Furthermore, long-term postoperative rehabilitation is essential. In addition, transplantation of cartilage tissue prepared from iPS cells has been studied, but it has not yet been clinically applied.

It is known that there are ever three subtypes of salt-induced kinase (SIK) belonging to an AMP-activated protein kinase family of serine-threonine kinases, SIK1 (SNF1LK), SIK2 (SNF1LK2, QIK, or KIAA0781), and SIK3 (QSK, KIAA0999, or FLJ12240) have been reported, respectively. Among them, regarding SIK3, it has been reported that suppressing the SIK3 signal suppresses maturation (hypertrophy) of chondrocytes in mice and suppresses development of osteoarthritis (Non Patent Literature 2). On the other hand, SIK2 has been ever known to be involved in cancer, diabetes, inflammatory diseases, neurodegenerative diseases, cardiovascular diseases, melanin formation, obesity, and the like (Patent Literatures 1 and 2, Non Patent Literature 1), its involvement in cartilage is unknown.

### CITATIONS LIST

### Patent Literatures

Patent Literature 1: WO 2004/018669 A
Patent Literature 2: WO 2014/093383 A

### Non Patent Literatures

Non Patent Literature 1: PlosONE, 2011, 6, e26148
Non Patent Literature 2: Nature Communications, 2016, 7, 10959

### SUMMARY OF INVENTION

### TECHNICAL PROBLEMS

An object of the present disclosure is to provide a novel therapeutic agent for a cartilage-related disease, having cartilage regenerating action.

### SOLUTIONS TO PROBLEMS

As a result of intensive studies to find a therapeutic agent for a cartilage-related disease, having cartilage regenerating action, the present inventors have found that a substance having SIK2 inhibitory action solves the above-mentioned problems by promoting differentiation of chondrocytes.

Therefore, in one aspect, the present disclosure provides a therapeutic agent for a cartilage-related disease, containing a substance having SIK2 inhibitory action.

In one aspect, the present disclosure provides a medicine for treatment of a cartilage-related disease, containing a substance having SIK2 inhibitory action in combination with at least one selected from a bone morphogenetic protein, an anti-inflammatory steroid, a non-steroidal anti-inflammatory drug, duloxetine, capsaicin, prostaglandin, a growth factor, a parathyroid hormone drug, a vitamin D derivative, a vitamin A derivative, a metalloproteinase inhibitor, a phosphodiesterase 4 inhibitor, an elastase inhibitor, an NFκB decoy oligo, a chondrocyte proliferation promoting factor, a chondroprotector, and an SIK3 inhibitor.

In one aspect, the present disclosure provides a therapeutic agent for a cartilage-related disease, containing at least one selected from a bone morphogenetic protein, an anti-inflammatory steroid, a non-steroidal anti-inflammatory drug, duloxetine, capsaicin, prostaglandin, a growth factor, a parathyroid hormone drug, a vitamin D derivative, a vitamin A derivative, a metalloproteinase inhibitor, a phosphodiesterase 4 inhibitor, an elastase inhibitor, an NFκB decoy oligo, a chondrocyte proliferation promoting factor, a chondroprotector, and an SIK3 inhibitor, in which the therapeutic agent is administered in combination with a substance having SIK2 inhibitory action.

In one aspect, the present disclosure provides a method for treating a cartilage-related disease, including administering an effective amount of a substance having SIK2 inhibitory action to a mammal.

In one aspect, the present disclosure provides use of a substance having SIK2 inhibitory action for producing a therapeutic agent for a cartilage-related disease.

In one aspect, the present disclosure provides a substance having SIK2 inhibitory action to be used to treat a cartilage-related disease.

In one aspect, the present disclosure is a method for screening a substance that promotes chondrocyte differentiation and/or has cartilage regenerating action, including:
(1) a step of measuring expression or function of SIK2 in the presence of a test substance, and
(2) a step of selecting a substance in which the expression or function of SIK2 is suppressed in step (1).

In one aspect, the present disclosure provides a chondrocyte differentiation promoter comprising a substance having SIK2 inhibitory action.

In one aspect, the present disclosure provides a cartilage regenerating agent containing a substance having SIK2 inhibitory action.

### ADVANTAGEOUS EFFECTS OF INVENTION

The present disclosure provides a new therapeutic agent for a cartilage-related disease.

### BRIEF DESCRIPTION OF DRAWINGS

Fig. 1 is a diagram showing comparison of SIK2 and SIK3 antisense oligonucleotide groups, with a Vehicle group as 100, for chondrocyte pellet size after culture in Example 1.
Fig. 2 is a diagram showing comparison of SIK2 and SIK3 antisense oligonucleotide groups, with a Vehicle group as 1, for a ratio of cartilage differentiation markers COL2A1/COL1A2 after culture in Example 1.

### DESCRIPTION OF EMBODIMENTS

In the present disclosure, the substance having SIK2 inhibitory action means a substance that suppresses expression or function of SIK2, and may be, for example, a low molecular weight compound, or a high molecular weight protein, polypeptide, polynucleotide (DNA, RNA, genes), antisense nucleic acid, decoy, antibody, plant extract, fermented product, vaccine, or the like. In addition, the substance having SIK2 inhibitory action in the present disclosure includes not only those found so far but also those found in the future.

Examples of the low molecular weight compound having SIK2 inhibitory action include large cyclic pyrazolopyrimidine and imidazopyridazine compounds represented formula (I) in WO 2014/140313 A, pyrrolopyridine or pyrazolopyridine compound represented by formula (I), (IA), or (IB) in Patent Literature 2, macrocyclic compounds represented by formula (I) in WO 2016/023014 A, imidazolyl compounds represented by formula (II), or urea and carbamate compounds represented by formula (III-A), flavonoid compounds containing 4'-O-methylfisetin described in Non Patent Literature 1, MRT67307, KIN112, and HG-9-91-01 described in PNAS, 2012,109,16986-16991, 6,6-bicyclic compounds described in ACS Chemical Biology, 2016, 11, 2105-2111, and the like.

The substance having SIK2 inhibitory action can be a SIK2 antisense nucleic acid. An antisense nucleic acid means a nucleic acid that inhibits its expression by hybridizing to mRNA and/or a gene. The antisense nucleic acid may be any of DNA, RNA or DNA/RNA chimera, and is preferably DNA. The antisense nucleic acid consists of, for example, 10 to 30, 12 to 25, 14 to 20, or 15 to 17 nucleotides.

The antisense nucleic acid may contain one or more artificial nucleotides. As the artificial nucleotide, one having a structure different from that of natural nucleotides and enhancing nuclease resistance and binding affinity with a target sequence may be selected. For example, artificial nucleotides described in Deleavey, G.F., & Damha, M.J. (2012). Designing chemically modified oligonucleotides for targeted gene silencing. Chemistry & biology, 19(8), 937-954, which are incorporated herein by reference, can be used. Examples of the artificial nucleotide include abasic nucleosides; arabinonucleosides, 2'-deoxyuridine, α-deoxyribonucleosides, β-L-deoxyribonucleosides, and other nucleosides with sugar modifications; peptide nucleic acids (PNAs), phosphate group-linked peptide nucleic acids (PHONA), cross-linked artificial nucleic acids (LNA), 2'-O,4'-C-ethylene cross-linked nucleic acids (ENA), constrained ethyl (cEt), morpholino nucleic acid, and the like. The artificial nucleotides with sugar modifications include those with sugar modifications of substituted pentasaccharides such as 2'-O-methylribose, 2'-O-methoxyethylribose, 2'-deoxy-2'-fluororibose, and 3'-O-methylribose; 1',2'-deoxyribose; arabinose; substituted arabinose sugars; hexamonosaccharides, and alpha-anomer. Examples of the artificial nucleotides with a modified base include those with pyrimidine such as 5-hydroxycytosine, 5-methylcytosine, 5-fluorouracil or 4-thiouracil; purine such as 6-methyladenine or 6-thioguanosine; other heterocyclic base, or the like. The artificial nucleotides in the antisense nucleic acid may all be of the same type, or may be two or more different types of artificial nucleotides.

The SIK2 antisense nucleic acid has a nucleotide sequence that hybridizes to SIK2 mRNA and/or gene. In one embodiment, the SIK2 antisense nucleic acid consists of a nucleotide sequence having at least about 60%, about 70%, about 80%, about 85%, about 90%, about 95%, about 96%, about 97%, about 98%, or about 99% or more sequence identity with any of sequences of SEQ ID NOs: 1 to 5. In another embodiment, the SIK2 antisense nucleic acid consists of a nucleotide sequence in which one, two or three nucleotides have been deleted, substituted, added or inserted in any of nucleotide sequences of SEQ ID NOs: 1 to 5. In one embodiment, the SIK2 antisense nucleic acid includes a nucleotide sequence having about 90% or more identity with any of SEQ ID NOs: 1 to 5. In one embodiment, the SIK2 antisense nucleic acid includes any of the nucleotide sequences of SEQ ID NOs: 1 to 5. In one embodiment, the SIK2 antisense nucleic acid consists of any of the nucleotide sequences of SEQ ID NOs: 1 to 5.

In one embodiment, the SIK2 antisense nucleic acid consists of a nucleotide sequence having at least about 60%, about 70%, about 80%, about 85%, about 90%, about 95%, about 96%, about 97%, about 98%, or about 99% or more sequence identity with a sequence of SEQ ID NO: 1 (ACAACAACTGCACTTT). In another embodiment, the SIK2 antisense nucleic acid consists of a nucleotide sequence in which one, two or three nucleotides have been deleted, substituted, added or inserted in a nucleotide sequence of SEQ ID NO: 1. In one embodiment, the SIK2 antisense nucleic acid includes a nucleotide sequence having about 90% or more identity with SEQ ID NO: 1. In one embodiment, the SIK2 antisense nucleic acid includes the nucleotide sequence of SEQ ID NO: 1. In one embodiment, the SIK2 antisense nucleic acid consists of the nucleotide sequence of SEQ ID NO: 1.

In the present disclosure, nucleotide sequence identity means a degree of similarity of sequences between oligonucleotides, and is determined by comparing two sequences aligned to an optimum state (a state in which nucleotide consensus is maximized) over a region of the sequences to be compared. Sequence identity number (%) can be calculated by determining the same nucleotides present in both sequences, determining a number of matching sites, then dividing the number of matching sites by a total number of nucleotides in the sequence region to be compared, and multiplying the obtained value by 100. Algorithms for obtaining optimal alignment and sequence identity include various algorithms commonly available to those skilled in the art (e.g., BLAST algorithm, FASTA algorithm, etc.). The sequence identity can be determined, for example, using sequence analysis software such as BLAST and FASTA.

In the present disclosure, cartilage-related diseases mean diseases accompanying a state in which cartilage is damaged (for example, deformation, wear, damage, defect, degeneration or rupture, etc.) or a state in which normal cartilage is not formed due to dyschondrogenesis or the like. Examples thereof include cartilage damage, articular disc damage, meniscus damage, osteoarthritis (for example, gonarthrosis, coxarthrosis, etc.), chondrodysplasia, achondroplasia, achondrogenesis, dyschondrogenesis, chondrodystrophia, articular chondrocalcinosis, disk herniation, keypuncher's disease, temporomandibular joint disease, cartilage defect, anotia, microtia, osteochondrosis, spondylosis deformans, osteochondral defect, osteomalacia, rickets, osteoporosis, renal osteodystrophy, Paget's disease of bone, repair and repair failure of fracture, refracture, osteosarcoma or myeloma. The cartilage-related diseases also include cartilage degeneration related to, caused by, or associated with a disease selected from rheumatoid arthritis, periarthritis scapulohumeralis, acute purulent arthritis, tuberculous arthritis, syphilitic arthritis, systemic lupus erythematosus, osteitis fibrosa, relapsing polychondritis, and osteochondritis dissecans.

In the present disclosure, treatment of cartilage-related diseases means treatment and/or prevention of cartilage-related diseases. The treatment of cartilage-related diseases includes regeneration of damaged cartilage, delay or stop of progression of cartilage disorder, or suppression of recurrence of cartilage disorder (prevention or delay of onset of cartilage disorder in patients previously suffered from cartilage disorders), ordinary cartilage formation, or improvement of at least one of one or more symptoms caused by cartilage disorder. Further, it is preferable that inflammation is secondarily subsided and a disease causing cartilage disorder is completely cured by improving the cartilage disorder. Prevention of cartilage-related diseases includes prevention or delay of the onset of cartilage disorder.

In one embodiment, the therapeutic agent of the present disclosure can regenerate cartilage. In the present disclosure, cartilage regeneration means regeneration of cartilage tissue, particularly cartilage tissue in articular cartilage, and may further also include maintenance of function of the regenerated cartilage tissue. Cartilage regeneration can be mediated by any action of chondrocyte proliferation promotion, chondrocyte differentiation promotion, suppression of chondrocalcinosis, or suppression of cartilage degradation, or a multiple combination thereof.

In the present disclosure, the chondrocyte differentiation promotion means that differentiation of cells in a differentiation stage prior to chondrocytes into chondrocytes is promoted, and can also further include that the differentiated chondrocytes form a cartilage tissue.

The cartilage differentiation promoting action of the therapeutic agent of the present disclosure can be utilized for promoting callus formation in fracture repair process. Therefore, the therapeutic agent of the present disclosure can also be used, for example, for the purpose of promoting fracture repair or treating fracture repair failure. Furthermore, the therapeutic agent of the present disclosure can also be used in carrying out surgical treatment aimed at treating cartilage-related diseases, for example, bone fenestration, osteochondral graft, chondrocyte transplantation and debridement, and can also be used in culturing chondrocytes for transplantation or transplanting the chondrocytes. In addition, the therapeutic agent of the present disclosure can be administered with chondrocytes for transplantation.

Subjects to which the therapeutic agent of the present disclosure are applied include mammals (pets, livestock, laboratory animals, etc.) including humans. For example, the subject can be human, dog, cat, rabbit, mouse, cow, pig, goat, sheep, horse, monkey, guinea pig, rat, mouse, and the like. In one embodiment, the subject is a human.

The therapeutic agent of the present disclosure may be administered as a combined agent, in combination with other surgical treatment and/or other agent, for example, for 1) complementing and/or enhancing the therapeutic effect of the therapeutic agent of the present disclosure, 2) improving dynamics and absorption of the therapeutic agent of the present disclosure, reducing a dose, and/or 3) reducing side effects of the therapeutic agent of the present disclosure.

When the therapeutic agent of the present disclosure is used together with surgical treatment, simultaneous administration and staggered administration are included. In the case of staggered administration, time after the surgical treatment is performed is not particularly limited.

The combined agent of the therapeutic agent of the present disclosure and the other agent may be administered in the form of a combination drug in which both components are mixed in one preparation, or may be administered in the form of separate preparations. When administered as separate preparations, simultaneous administration and staggered administration are included. Further, for the staggered administration, the therapeutic agent of the present disclosure may be administered first and the other agent may be administered later, or the other agent may be administered first and the therapeutic agent of the present disclosure may be administered later. Each administration method may be the same or different.

Examples of the agent to be combined with the therapeutic agent of the present disclosure include a bone morphogenetic protein, an anti-inflammatory steroid, a non-steroidal anti-inflammatory drug, duloxetine, capsaicin, prostaglandin, a growth factor, a parathyroid hormone drug, a vitamin D derivative, a vitamin A derivative, a metalloproteinase inhibitor, a phosphodiesterase 4 inhibitor, an elastase inhibitor, an NFκB decoy oligo, a chondrocyte proliferation promoting factor, a chondroprotector, an SIK3 inhibitor, and the like.

Examples of the bone morphogenetic protein include YM484 (BMP-2), BMP-7, and the like.

Examples of the anti-inflammatory steroid include amcinonide, hydrocortisone sodium succinate, prednisolone sodium succinate, methylprednisolone sodium succinate, ciclesonide, difluprednate, betamethasone propionate, dexamethasone, dexamethasone palmitate, deflazacort, triamcinolone, triamcinolone acetonide, halcinonide, dexamethasone palmitate, hydrocortisone, flumetasone pivalate, prednisolone butylacetate, budesonide, prasterone sulfate, mometasone furancarbonate, fluocinonide, fluocinolone acetonide, fludroxycortide, flunisolide, prednisolone, alclometasone propionate, clobetasol propionate, dexamethasone propionate, deprodone propionate, fluticasone propionate, beclometasone propionate, betamethasone, methylprednisolone, methylprednisolone suleptanate, methylprednisolone sodium succinate, mometasone furancarbonate, dexamethasone sodium phosphate, hydrocortisone sodium phosphate, prednisolone sodium phosphate, diflucortolone valerate, dexamethasone valerate, betamethasone valerate, prednisolone valerate acetate, cortisone acetate, diflorasone acetate, dexamethasone acetate, triamcinolone acetate, paramethasone acetate, halopredone acetate, fludrocortisone acetate, prednisolone acetate, methylprednisolone acetate, clobetasone butyrate, hydrocortisone butyrate, hydrocortisone butyrate propionate, betamethasone butyrate propionate, ST-126P, and the like.

Examples of the non-steroidal anti-inflammatory drug include aspirin, aspirin dialuminate, acemetacin, alminoprofen, ampiroxicam, amfenac sodium, ibuprofen, ibuprofenpiconol, indomethacin, indomethacin farnesil, ufenamate, etodolac, epirizole, emorfazone, oxaprozin, oxyphenbutazone, sulindac, ketophenylbutazone, ketoprofen, sasapyrine, sodium salicylate, isopropylantipyrine, zaltoprofen, diclofenac sodium, diflunisal, dimethylisopropylazulene, suprofen, tiaprofenic acid, tenoxicam, tolfenamic acid, tolmetin sodium, nabumetone, naproxen, piroxicam, fenoprofen calcium, felbinac, fenbufen, bufexamac, pranoprofen, flurbiprofen, flurbiprofen axetil, floctafenine, proglumetacin, proglumetacin maleate, mefenamic acid, aluminum mefenamate, mofezolac, loxoprofen sodium, tiaramide hydrochloride, tinoridine hydrochloride, sulpyrine, migrenin, acetaminophen, phenacetin, dimetotiazine mesilate, simetride combination drug, isopropylantipyrine, and the like.

Examples of the prostaglandin include PG receptor agonists, PG receptor antagonists, and the like.

Examples of the growth factor include fibroblast growth factors (FGF), vascular endothelial growth factors (VEGF), hepatocyte growth factors (HGF), insulin-like growth factors, and the like.

Examples of the parathyroid hormone drug include teriparatide (acetate) and the like.

Examples of the vitamin D derivative include alfacalcidol, falecalcitriol, calcitriol, 1α,25-dihydroxycholecalciferol, dihydrotachysterol, ST-630, KDR, ST-630, ED-71, rocaltrol (Ro44-7190), and the like.

Examples of the vitamin A derivative include retinol, retinal, retinoic acid, retinol palmitate, retinol acetate, cylasphere retinol, pure retinol, tocopheryl retinoate, and the like.

Examples of the metalloproteinase inhibitor include marimastat, neovastat, BMS-275291, prinomastat, metastat, S-3304, and the like.

Examples of the phosphodiesterase 4 (PDE4) inhibitor include cilomilast, roflumilast, arofylline, OPC-6535, IC-485, AWD-12-281, CC-10004, CC-1088, KW-4490, Iirimilast, ZK-117137, YM-976, BY-61-9987, CC-7085, CDC-998, MEM-1414, ND-1251, Bay19-8004, D-4396, PD-168787, atizoram, cipamfylline, rolipram, NIK-616, SCH-351591, V-11294A, and the like.

Examples of the elastase inhibitor include sivelestat sodium (hydrate) and the like.

Examples of the chondrocyte proliferation promoting factor include transforming growth factors-β (TGF-β), insulin-like growth factors (IGF-I), basic fibroblast growth factors (bFGF), combination of epidermal growth factor (EGF) and insulin, growth hormones (GH), platelet-derived growth factors (PDGF), and the like.

Examples of the chondroprotector include hyaluronic acid preparations (e.g., Artz, Suvenyl, Synvisc, sodium hyaluronate, etc.), glucosamine, chondroitin sulfate, polysulfated glycosaminoglycan (e.g., chondroitin 4-sulfate, chondroitin 6-sulfate, dermatan sulfate, heparan sulfate, heparin, etc.), and the like.

The SIK3 inhibitor may be, for example, a substance that suppresses expression or function of SIK3, and examples thereof include low molecular weight compounds such as pterosin B and a SIK3 antisense nucleic acid.

In one embodiment, the SIK3 antisense nucleic acid has a nucleotide sequence that hybridizes to SIK3 mRNA and/or gene. In one embodiment, the SIK2 antisense nucleic acid consists of a nucleotide sequence having at least about 60%, about 70%, about 80%, about 85%, about 90%, about 95%, about 96%, about 97%, about 98%, or about 99% or more sequence identity with a sequence of SEQ ID NO: 6 (GGTTAGTGCCACGTTG). In another embodiment, the SIK3 antisense nucleic acid consists of a nucleotide sequence in which one, two or three nucleotides have been deleted, substituted, added or inserted in a nucleotide sequence of SEQ ID NO: 6. In one embodiment, the SIK3 antisense nucleic acid includes a nucleotide sequence having about 90% or more identity with SEQ ID NO: 6. In one embodiment, the SIK3 antisense nucleic acid includes the nucleotide sequence of SEQ ID NO: 6. In one embodiment, the SIK3 antisense nucleic acid consists of the nucleotide sequence of SEQ ID NO: 6.

The blending ratio of the therapeutic agent of the present disclosure and the other drug can be appropriately selected depending on an age and a weight of an administration subject, an administration method, an administration time, and the like. For example, 0.01 to 100 parts by weight of other agent may be used with respect to 1 part by weight of the therapeutic agent of the present disclosure.

The other agent may be administered by combining arbitrary two or more types.

In addition, the other agent includes not only those found so far but also those found in the future, based on the above-mentioned mechanism.

In order to use the therapeutic agent of the present disclosure as a single agent or as a combined agent in combination with other agent for the purpose of treating the diseases, a substance having SIK2 inhibitory action, which is an active ingredient, is usually formulated with a pharmaceutically acceptable carrier such as various additives or solvent and then administered systemically or topically in oral or parenteral form. Here, the pharmaceutically acceptable carrier means a substance other than the active ingredient, which is generally used in preparation of pharmaceutical product. The pharmaceutically acceptable carrier preferably has no pharmacological action at a dose of the preparation, is harmless, and does not interfere with a therapeutic effect of the active ingredient. In addition, the pharmaceutically acceptable carrier can also be used for the purpose of enhancing usefulness of the active ingredient and the preparation, facilitating formulation, stabilizing quality, improving usability, and the like. Specifically, substances such as those described in "Encyclopedia of Pharmaceutical Additives" published by Yakuji Nippo, Limited in 2000 (edited by International Pharmaceutical Excipients Council Japan) or the like may be appropriately selected according to the purpose.

A dose of the therapeutic agent of the present disclosure varies depending on the substance having SIK2 inhibitory action used, and at the same time, varies depending on an age, a weight, symptom, a therapeutic effect, an administration method, a treatment time and the like, but the therapeutic agent of the present disclosure is administered orally, usually, in a range of 1 µg to 100 mg per once per adult, once to several times a day. Alternatively, the therapeutic agent of the present disclosure is administered parenterally (e.g., intra-articularly), in a range of 0.1 ng to 10 mg per once per adult, once to several times a day, or continuously administered intravenously, in a range of 1 to 24 hours a day. In the case of a prolonged-release preparation such as implants/pellets or a prolonged-release injection described later, it is administered parenterally in a range of once every few days to once every two months. As described above, since the dose varies depending on various conditions, a dose smaller than the above dose may be sufficient, or administration beyond the range may be necessary.

Examples of a dosage form used for administration include preparations for oral administration (e.g., tablets, capsules, granules, powders, liquids and solutions for oral administration, syrups, jellies for oral administration, etc.), preparations for oro-mucosal application (e.g., tablets for oro-mucosal application, sprays for oro-mucosal application, semi-solid preparations for oro-mucosal application, preparations for gargles, etc.), preparations for injection (e.g. injections, etc.), preparations for dialysis (e.g. dialysis agents, etc.), preparations for inhalation (e.g., inhalants, etc.), preparations for ophthalmic application (e.g. ophthalmic liquids and solutions, ophthalmic ointments, etc.), preparations for otic application (e.g. ear preparations, etc.), preparations for nasal application (e.g. nasal preparations, etc.), preparations for rectal application (e.g. suppositories for rectal application, semi-solid preparations for rectal application, enemas for rectal application, etc.), preparations for vaginal application (e.g. tablets for vaginal use, suppositories for vaginal use, etc.), preparations for cutaneous application (e.g. solid preparations for topical application, liquids and solutions for topical application, sprays, ointments, creams, gels, patches, etc.), and the like.

### [Preparations for oral administration]

Examples of the preparations for oral administration include tablets, capsules, granules, powders, liquids and solutions for oral administration, syrups, jellies for oral administration, and the like. In addition, preparations for oral administration include fast-disintegrating preparations in which release of active ingredient(s) from the preparation is not particularly adjusted, and release-controlled preparations in which release is adjusted according to the purpose by a unique formulation design and manufacturing method, for example, enteric-coated preparations, sustained-release preparations, and the like. The enteric-coated preparation refers to a preparation designed so that active ingredient(s) is not released in stomach but mainly in small intestine for the purpose of preventing decomposition of the active ingredient(s) in the stomach or reducing an irritating effect of the active ingredient(s) on the stomach, and can be usually produced by applying a film using an acid-insoluble enteric base. The sustained-release preparation refers to a preparation in which a release rate, release time, and release site of active ingredient(s) from the preparation are adjusted for the purpose of reducing the number of administrations, reducing side effects or the like, and can be usually produced by using an appropriate sustained-release agent. Of the preparations for oral administration, capsules, granules, tablets, etc. can be also coated with appropriate coating agents such as sugars, sugar alcohols or polymer compounds, for the purpose of facilitating administration or preventing decomposition of the active ingredient.

### (1) Tablets

Tablets are solid preparations having a certain shape and size, intended for oral administration, and intraoral quickly disintegrating tablets, chewable tablets, effervescent tablets, dispersible tablets, soluble tablets and the like are included besides those generally called tablets such as uncoated tablets, film-coated tablets, sugar-coated tablets, multi-layered tablets, and dry-coated tablets. Uncoated tables are usually produced using the following method (a), (b) or (c):
(a) Mix homogeneously active ingredient(s) with additives such as excipients, binders and disintegrants, granulate the mixture with water or a solution containing a binder by an appropriate method, then mix with a lubricant and the like, and compression-mold the mixture;
(b) Mix homogeneously active ingredient(s) with additives such as excipients, binders and disintegrants, and directly compression-mold the mixture, or mix homogeneously granules previously prepared with additives with active ingredient(s), a lubricant and the like, and then compression-mold the mixture; and
(c) Mix homogeneously active ingredient(s) with additives such as excipients and binders, pour the kneaded product moistened with a solvent into a certain mold for molding, and then dry the molded product by an appropriate method.

Film-coated tablets can be usually produced by coating uncoated tablets with thin films using appropriate coating agents such as polymer compounds. Sugar-coated tablets can be usually produced by coating uncoated tablets using coating agents including sugars or sugar alcohols. Multi-layer tablets can be produced by stacking particulates having different compositions in layers and compression-molding them by an appropriate method. Dry-coated tablets can be produced by covering inner core tablets with outer layers having different compositions. In addition, tablets can be also prepared as enteric coated tablets or sustained-release tablets using an appropriate known method. Intraoral quickly disintegrating tablets, chewable tablets, effervescent tablets, dispersible tablets and soluble tablets are tablets that have been given unique functions by appropriate selection of additives, and can be produced according to the method for producing tablets described above. Intraoral quickly disintegrating tablets refer to tablets that can be quickly dissolved or disintegrated in the oral cavity and taken; chewable tablets refer to tablets that are chewed and taken; effervescent tablets refer to tablets that are dissolved or dispersed while rapidly bubble in water; dispersible tablets refer to tablets that are dispersed in water and taken; and soluble tablets refer to tablets that are dissolved in water and taken. Effervescent tablets can be produced by using an appropriate acidic substance, carbonate, hydrogen carbonate or the like as additives.

### (2) Capsules

Capsules are preparations filled in capsules or encapsulation-molded with capsule bases, and hard capsules, soft capsules and the like are included. Hard capsules can be produced by a method where a homogeneous mixture obtained by mixing active ingredient(s) with additives such as excipients, or granules or molded product obtained by an appropriate method, are filled into capsule as they are or after slightly molding. Soft capsules can be produced by a method where a mixture obtained by adding additives to active ingredient(s) is encapsulation-molded in a certain shape with appropriate capsule bases such as gelatin plasticized by addition of glycerin, D-sorbitol, or the like. Capsules can be prepared as enteric-coated capsules or extended-release capsules using an appropriate known method, and a coloring agent, a preservative or the like can also be added to the capsule base.

### (3) Granules

Granules are preparations granulated into granules, and effervescent granules and the like are included besides those generally called granules. Granules are usually produced using the following method (a), (b) or (c):
(a) Mix homogeneously powdered active ingredient(s) with an excipient, a binder, a disintegrant or other additives, and then granulate the mixture by an appropriate method;
(b) Mix homogeneously active ingredient(s) previously prepared in granules with additives such as excipients; and
(c) Mix active ingredient(s) previously prepared in granules with additives such as excipients, and granulate the mixture by an appropriate method.

Granules can be also coated if necessary, and can be also prepared as enteric-coated granules or extended-release granules using an appropriate known method. Effervescent granules can be produced by using an appropriate acidic substance, carbonate, hydrogen carbonate or the like as additives. The effervescent granules refer to granules that are dissolved or dispersed while rapidly bubble in water. Granules can be also prepared as fine granules by adjusting particle size.

### (4) Powders

Powders are preparations in powder form, and can be usually produced by mixing homogeneously active ingredient(s) with an excipient or other additives.

### (5) Liquids and solutions for oral administration

Liquids and solutions for oral administration are liquid, or flowable, viscous and gelatinous preparations, and elixirs, suspensions, emulsions and lemonades and the like are included besides those generally called oral solutions. Liquids and solutions for oral administration can be usually produced by mixing active ingredient(s) with additives and purified water, homogeneously dissolving, emulsifying or suspending the mixture, and filtering the mixture if necessary. Elixirs refer to clear, sweetened and aromatic liquids and solutions for oral administration in liquid form containing ethanol, and can be usually produced by adding ethanol, purified water, an aromatic agent and sucrose, other sugars or sweetening agents to solid active ingredient(s) or exudate thereof and dissolving them, and clarifying the solution by filtration or other methods. Suspensions refer to liquids and solutions for oral administration of active ingredient(s) finely and homogeneously suspended, and can be usually produced by adding a suspending agent or other additives and purified water or oil to a solid active ingredient(s) and suspending them by an appropriate method to homogenize the entire suspension. Emulsions refer to liquids and solutions for oral administration of active ingredient(s) finely and homogeneously emulsified, and can be usually produced by adding an emulsifying agent and purified water to liquid active ingredient(s) and emulsifying them by an appropriate method to homogenize the entire suspension. In addition, lemonades refer to sweet and sour, clear liquids and solutions for oral administration in liquid form.

### (6) Syrups

Syrups are viscous liquid or solid preparations containing sugars or sweetening agents, and preparations for syrups and the like are included. Syrups can be usually produced by adding active ingredient(s) to a solution of syrup, other sugars or sweetening agents, or a simple syrup, and dissolving, mixing, suspending or emulsifying them, and if necessary, boiling and then filtering the mixture while hot. Preparations for syrups refer to preparations in form of granules or powders, which become syrups by adding water, and they may be also termed dry syrups. Preparations for syrups can be usually produced by using sugars or sweetening agents as additives, according to the production method of granules or powders.

### (7) Jellies for oral administration

Jellies for oral administration are non-flowable molded gelatinous preparations, and can be usually produced by mixing active ingredient(s) with additives and a polymer gel base, and gelatinizing and molding the mixture into a certain shape by an appropriate method.

### [Preparations for injection]

### (1) Injections

Injections are sterile preparations to be administered subcutaneously, intramuscularly, intraarticularly, or directly into body tissues or organs such as blood vessels, in form of a solution, a suspension or an emulsion, or of a solid to be dissolved or suspended before use, and freeze-dried injections, powders for injections, prefilled syringes for injections, cartridges for injections, parenteral infusions, implants/pellets, prolonged-release injections and the like are included. Injections are usually produced using the following method (a) or (b):
(a) Dissolve, suspend or emulsify homogeneously active ingredient(s) as it is or added with additives in water for injection, other aqueous solvent or non-aqueous solvent or the like, fill the mixture into a container for injection, seal, and sterilize; and
(b) Dissolve, suspend or emulsify homogeneously active ingredient(s) as it is or added with additives in water for injection, other aqueous solvent or non-aqueous solvent or the like and filtrate aseptically the mixture, or prepare aseptically a homogeneous liquid, fill the mixture into a container for injection, and seal.

Freeze-dried Injections can be usually produced by dissolving active ingredient(s) as it is or active ingredient(s) with additives such as excipients in water for injection, filtrate aseptically the solution, filling the filtrate into a container for injection and then being freeze-dried, or freeze-drying the filtrate in special containers and then filling it into individual containers for injection. Powders for injections can be usually produced by filtrating aseptically active ingredient(s), adding powders obtained by crystallization or the powders added with sterilized additives, and filling the powders into a container for injection. Prefilled syringes for injections can be usually produced by filling into syringes active ingredient(s) as it is or a solution, suspension, or emulsion prepared using active ingredient(s) and additives. Cartridges for injections refer to injections used by fixing a cartridge filled with a chemical solution in an injector for exclusive use. The cartridge filled with a chemical solution can be usually produced by filling into a cartridge active ingredient(s) as it is or a solution, suspension, or emulsion prepared using active ingredient(s) and additives. Parenteral infusions usually refer to injections of 100 mL or more, intended for intravenous administration. Implants/pellets refer to solid or gel-like injections applied subcutaneously, intramuscularly, etc. using an implant device or by means of operative treatment, for the purpose of releasing active ingredient(s) for a long period of time. Implants/pellets can be usually produced in a form of pellet, microsphere or gel using biodegradable polymer compounds. Prolonged release injections refer to injections to be applied intramuscularly or intraarticularly, for the purpose of releasing active ingredient(s) for a long period of time, and can be usually produced by dissolving or suspending active ingredient(s) in vegetable oil or the like, or by preparing a suspension of microspheres using biodegradable polymer compounds.

### [Preparations for cutaneous application]

### (1) Solid preparations for topical application

Solid preparations for topical application are solid preparations intended for application or spraying on the skin including scalp or nails, and powders for topical application and the like are included. Powders for topical application are powdery solid preparations for topical application, and can be usually produced by mixing homogeneously active ingredient(s) with additives such as excipients and then pulverizing the mixture.

### (2) Liquids and solutions for topical application

Liquids and solutions for topical application are liquid preparations intended for application to the skin including scalp or nails, and liniments, lotions and the like are included. Liquids and solutions for topical application are usually prepared by mixing active ingredient(s) with a solvent, additives and the like, dissolving, emulsifying or suspending the mixture, and filtering the mixture if necessary. Liniments are liquid or muddy preparations for topical application to the skin by rubbing. Lotions refer to liquids and solutions for topical application in which active ingredient(s) are dissolved, emulsified or finely dispersed in an aqueous liquid, and can be usually produced by preparing a solution, suspension, or emulsion using active ingredient(s), additives and purified water, and homogenizing the entire mixture.

### (3) Sprays

Sprays are preparations intended for spraying active ingredient(s) onto the skin in mists, powders, foams, paste state, etc. and aerosols for topical application and pump sprays and the like are included. Sprays can be usually produced by preparing a solution or suspension of active ingredient(s), filtering it if necessary, and then filling it into a container. Aerosols for topical application are sprays which atomize active ingredient(s) together with liquefied or compressed gas filled in a container. Aerosols for topical application can be usually produced by preparing a solution or suspension of active ingredient(s), filling it with a liquefied propellant in a pressure-resistant container, and setting a continuous spray valve. Additives such as a dispersing agent and a stabilizer can be added to the aerosols for topical application, if necessary. Pump sprays are sprays which atomize active ingredient(s) in a container by pumping. Pump sprays can be usually produced by dissolving or suspending active ingredient(s) and additives, and setting a pump to the filled container.

### (4) Creams

Creams are semi-solid preparations intended for application to the skin, which are in the form of oil-in-water or water-in-oil emulsions, and hydrophobic preparations in the form of water-in-oil emulsions may be also termed oily creams. Creams can be usually produced by using vaseline, higher alcohol or the like as it is or added with additives such as an emulsifier as an oil phase, separately using purified water as it is or added with additives such as an emulsifier as an aqueous phase, adding active ingredient(s) to either phase, heating each of them, and stirring and emulsifying the oil phase and the aqueous phase together until the entire mixture is homogeneous.

### (5) Gels

Gels are gelatinous preparations intended for application to the skin, and aqueous gels, oily gels and the like are included. Aqueous gels can be produced by adding a polymer compound, other additives and purified water to active ingredient(s), and dissolving or suspending them, and heating and cooling the mixture, or adding a gelatinizing agent for crosslinking. Oily gels can be produced by mixing a liquid oily base such as glycols and higher alcohols and other additives with active ingredient(s).

### (6) Patches

Patches are preparations intended to be attached on the skin, and tapes/plasters and cataplasms/gel patches and the like are included. Patches can usually be produced by using a polymer compound or a mixture thereof as a base, mixing active ingredient(s) with the base to make it homogeneous, and spreading the mixture on a support or liner (peeling body) for molding. Percutaneous absorption type preparations can be also prepared by using a release rate-controlling membrane. If necessary, additives such as adhesive agents or penetration enhancers can be used in patches. Tapes refer to patches which use a base containing almost no water, and plasters, ointments and the like are included. Tapes can be usually produced by mixing homogeneously active substance(s) as it is or added with additives and a base of non water-soluble natural or synthetic polymer compounds such as resins, plastics or rubber, and spreading the mixture on a cloth or spreading or sealing the mixture on a plastic film or the like for molding. Tapes can be also produced by sealing a mixture of active substance(s) and a base with or without other additives in releasers composed with a release-controlling membrane, a support and a liner (peeling body) for molding. Cataplasms/Gel Patches refer to patches using bases containing water, and can be usually produced by mixing homogeneously active substance(s) with a liquid substance such as purified water or glycerin, or by mixing and kneading natural or synthetic polymers such as water soluble polymers and water absorbing polymers, with purified water, adding active substance(s), mixing homogeneously the entire mixture, and spreading the mixture on a cloth or the like for molding.

In another aspect, the present disclosure provides a method for treating a cartilage-related disease, including administering an effective amount of a substance having SIK2 inhibitory action to a mammal.

In yet another aspect, the present disclosure provides use of a substance having SIK2 inhibitory action for treatment of cartilage-related diseases.

In yet another aspect, the present disclosure provides a substance having SIK2 inhibitory action to be used to treat a cartilage-related disease.

In yet another aspect, the present disclosure provides use of a substance having SIK2 inhibitory action for producing a therapeutic agent for a cartilage-related disease.

In another aspect, the present disclosure provides a chondrocyte differentiation promoter containing a substance having SIK2 inhibitory action.

In yet another aspect, the present disclosure provides a method for promoting chondrocyte differentiation, including administering an effective amount of a substance having SIK2 inhibitory action to a mammal.

In yet another aspect, the present disclosure provides use of a substance having SIK2 inhibitory action for promotion of chondrocyte differentiation.

In yet another aspect, the present disclosure provides a substance having SIK2 inhibitory action, which is used for promoting chondrocyte differentiation.

In yet another aspect, the present disclosure provides use of a substance having SIK2 inhibitory action for producing a chondrocyte differentiation promoter.

In yet another aspect, the present disclosure provides a method for promoting chondrocyte differentiation, including treating chondrocyte progenitor cells with a substance having SIK2 inhibitory action in vitro.

In another aspect, the present disclosure provides a cartilage regenerating agent containing a substance having SIK2 inhibitory action.

In yet another aspect, the present disclosure provides a method for regenerating cartilage, including administering an effective amount of a substance having SIK2 inhibitory action to a mammal.

In yet another aspect, the present disclosure provides use of a substance having SIK2 inhibitory action for cartilage regeneration.

In yet another aspect, the present disclosure provides a substance having SIK2 inhibitory action, which is used for cartilage regeneration.

In yet another aspect, the present disclosure provides use of a substance having SIK2 inhibitory action for producing a cartilage regenerating agent.

In one aspect, the present disclosure provides a method for screening a substance that promotes chondrocyte differentiation and/or a substance that has cartilage regenerating action. Specifically, in step (1), expression or function of SIK2 is measured in the presence of a test substance, and in step (2), a test substance in which the expression or function of SIK2 is suppressed in step (1) is selected.

The "test substance" includes all substances such as polypeptides, proteins, polypeptides, amino acids, nucleic acids, polynucleotides (DNA, RNA, genes), antisense nucleic acids, decoys, lipids, sugars, low molecular weight compounds, antibodies, plant extracts, and fermented products. As the test substance, purified and isolated one can be typically used, but an unpurified or unisolated crude product or a living cell that produces the test substance may be used. The test substance may be provided in the form of a compound library, a nucleic acid library, a random peptide library, or the like, or may be provided as a natural product.

In step (1), a method of measuring the expression or function of SIK2 can be carried out, for example, by (1) measurement/analysis of degree of phosphorylation of a SIK2 substrate, (2) confirmation/analysis of a binding state with a SIK2 substrate, (3) measurement/analysis of degree of SIK2 protein amount or mRNA amount in SIK2-expressing cells, (4) measurement/analysis of degree of SIK2 promoter activity in SIK2-expressing cells, or (5) measurement/analysis of SIK2 signaling state, of the test substance.

Any of the measurement methods or analysis methods shown in (1) to (5) above can be performed by a known method.

Whether or not the test substance suppressed the expression or function of SIK2 can be determined by comparing with a measurement result of a negative control using no test substance. It may be compared with a measurement result of a positive control using a known substance having SIK2 inhibitory action (for example, an antisense nucleic acid having a nucleotide sequence of SEQ ID NO: 1).

The substances obtained by the screening method of the present disclosure can be used for promoting chondrocyte differentiation or cartilage regeneration. In addition, it can be selected as an active ingredient of a therapeutic agent for a cartilage-related disease.

The present disclosure provides, for example, the following embodiments.
[1] A therapeutic agent for a cartilage-related disease, containing a substance having SIK2 inhibitory action;
[2] The therapeutic agent according to [1] above, wherein the substance having SIK2 inhibitory action is a SIK2 antisense nucleic acid;
[3] The therapeutic agent according to [2] above, wherein the SIK2 antisense nucleic acid includes a nucleotide sequence having 90% or more identity with any of nucleotide sequences of SEQ ID NOs: 1 to 5;
[4] The therapeutic agent according to [2] or [3] above, wherein the SIK2 antisense nucleic acid includes any of the nucleotide sequences of SEQ ID NOs: 1 to 5;
[5] The therapeutic agent according to the above [2] or [3], wherein the SIK2 antisense nucleic acid includes a nucleotide sequence having 90% or more identity with the nucleotide sequence of SEQ ID NO: 1;
[6] The therapeutic agent according to any one of [2] to [5] above, wherein the SIK2 antisense nucleic acid includes the nucleotide sequence of SEQ ID NO: 1;
[7] The therapeutic agent according to any one of [1] to [6] above, wherein the cartilage-related disease is cartilage damage, articular disc damage, meniscus damage, osteoarthritis, chondrodysplasia, achondroplasia, achondrogenesis, dyschondrogenesis, chondrodystrophia, articular chondrocalcinosis, disk herniation, keypuncher's disease, temporomandibular joint disease, cartilage defect, anotia, microtia, osteochondrosis, spondylosis deformans, osteochondral defect, osteomalacia, rickets, osteoporosis, renal osteodystrophy, Paget's disease of bone, repair and repair failure of fracture, refracture, osteosarcoma or myeloma;
[8] The therapeutic agent according to any one of [1] to [6] above, wherein the cartilage-related disease is cartilage degeneration associated with a disease selected from rheumatoid arthritis, periarthritis scapulohumeralis, acute purulent arthritis, tuberculous arthritis, syphilitic arthritis, systemic lupus erythematosus, osteitis fibrosa, relapsing polychondritis, and osteochondritis dissecans;
[9] The therapeutic agent according to any one of [1] to [8] above, for regenerating cartilage;
[10] A medicine for treatment of a cartilage-related disease, containing a substance having SIK2 inhibitory action in combination with at least one selected from a bone morphogenetic protein, an anti-inflammatory steroid, a non-steroidal anti-inflammatory drug, duloxetine, capsaicin, prostaglandin, a growth factor, a parathyroid hormone drug, a vitamin D derivative, a vitamin A derivative, a metalloproteinase inhibitor, a phosphodiesterase 4 inhibitor, an elastase inhibitor, an NFκB decoy oligo, a chondrocyte proliferation promoting factor, a chondroprotector, and an SIK3 inhibitor;
[11] The therapeutic agent according to any one of [1] to [9] above, wherein the therapeutic agent is administered in combination with at least one selected from a bone morphogenetic protein, an anti-inflammatory steroid, a non-steroidal anti-inflammatory drug, duloxetine, capsaicin, prostaglandin, a growth factor, a parathyroid hormone drug, a vitamin D derivative, a vitamin A derivative, a metalloproteinase inhibitor, a phosphodiesterase 4 inhibitor, an elastase inhibitor, an NFκB decoy oligo, a chondrocyte proliferation promoting factor, a chondroprotector, and an SIK3 inhibitor;
[12] A therapeutic agent for a cartilage-related disease, containing at least one selected from a bone morphogenetic protein, an anti-inflammatory steroid, a non-steroidal anti-inflammatory drug, duloxetine, capsaicin, prostaglandin, a growth factor, a parathyroid hormone drug, a vitamin D derivative, a vitamin A derivative, a metalloproteinase inhibitor, a phosphodiesterase 4 inhibitor, an elastase inhibitor, an NFκB decoy oligo, a chondrocyte proliferation promoting factor, a chondroprotector, and an SIK3 inhibitor, wherein the therapeutic agent is administered in combination with a substance having SIK2 inhibitory action;
[13] A method for treating a cartilage-related disease, including administering an effective amount of a substance having SIK2 inhibitory action to a mammal;
[14] A use of a substance having SIK2 inhibitory action for producing a therapeutic agent for a cartilage-related disease;
[15] A substance having SIK2 inhibitory action to be used to treat a cartilage-related disease;
[16] A method for screening a substance that promotes chondrocyte differentiation and/or has cartilage regenerating action, including:
   (1) a step of measuring expression or function of SIK2 in the presence of a test substance, and
   (2) a step of selecting a test substance in which the expression or function of SIK2 is suppressed in step (1);
[17] A cartilage differentiation promoter containing a substance having SIK2 inhibitory action; and
[18] A cartilage regenerating agent containing a substance having SIK2 inhibitory action.

All documents cited herein are incorporated herein by reference.

All of the above description is non-limiting, and the present invention is defined in the appended claims and various modifications can be made without departing from the technical idea thereof. Hereinafter, the present invention will be described in detail with reference to examples, but the present invention is not limited thereto.

### EXAMPLES

### Example 1: SIK2 mRNA inhibitory effect of SIK2 antisense oligonucleotide

When SIK2 mRNA inhibitory effect of antisense oligonucleotides shown in the table below was measured, they all showed 70% or more inhibition.

**[Table 1]**

| | Nucleotide sequence | Sequence number |
|---|---|---|
| SIK2 ASO 1 | ACAACAACTGCACTTT | 1 |
| SIK2 ASO 2 | AACCGGCCATGATTAG | 2 |
| SIK2 ASO 3 | CGATGGATGGCTCATT | 3 |
| SIK2 ASO 4 | GACAATAATCAACAGC | 4 |
| SIK2 ASO 5 | TCAACAGCAGACAGGA | 5 |

### Example 2: Evaluation of chondrocyte differentiation promoting action using SIK2 and SIK3 antisense oligonucleotides

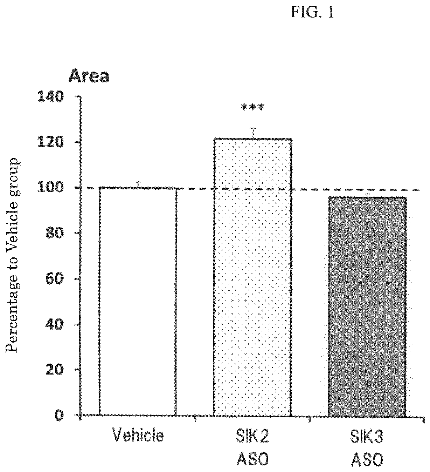

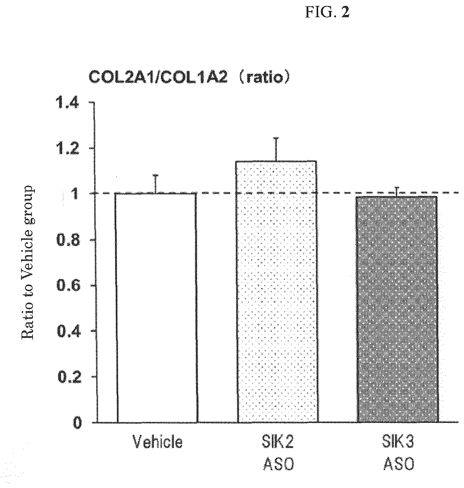

Normal human knee joint chondrocytes (Lonza Walkersville, Inc.) were thawed and cultured (culture conditions; 5% CO₂, 37°C, 95% Air, the same conditions applies hereafter.) in a subculture medium (DMEM/F-12 + glutaMAX, included in set of chondrocyte addition factors (Lonza Walkersville, Inc.)). Diluted subculture was performed after subconfluent, and two days after subculture, the culture was treated with antisense oligonucleotides against SIK2 and SIK3 (hereinafter SIK2 ASO; ACAACAACTGCACTTT (SEQ ID NO: 1) and SIK3 ASO; GGTTAGTGCCACGTTG (SEQ ID NO: 6)) in the presence of calcium chloride (9 mmol/L) (final concentration 1000 nmol/L). The next day, the medium was replaced with a subculture medium and cultured overnight, and the cultured cells two days after the introduction of SIK2 ASO and SIK3 ASO were seeded on a 96-well low adsorption culture plate at a density of 1.0 × 10⁵ cells/well, and pellet-cultured for 10 days. Chondrocyte pellets were imaged using Opera Phenix High Content Screening System (PerkinElmer), and pellet size (area of the pellet image) was calculated using an attached analysis software Harmony. The chondrocyte pellets after imaging were frozen in liquid nitrogen, crushed using AUTOMILL (Tokken, inc.), and then total RNA was extracted using RNeasy Plus Mini Kit (QIAGEN N.V.). cDNA was synthesized from a total RNA solution using High Capacity cDNA Reverse Transcription Kit, and expression level of each gene was measured by real-time polymerase chain reaction (PCR) method using TaqMan Fast Universal PCR Master Mix (2X) (Life Technologies Corporation) and Primer-Probe (COL2A1 assay ID: Hs01060345_m1, COL1A2 assay ID: Hs01028970_m1) of each gene. For pellet size and COL2A1/COL1A2, comparison between a Vehicle group, a SIK2 ASO group and a SIK3 ASO group was performed by t-test. The results are shown in Figs. 1 and 2.

As a result of analysis, SIK2 ASO treatment significantly increased the chondrocyte pellets. In addition, the cartilage differentiation markers COL2A1/COL1A2 increased.

From the above, a substance having SIK2 inhibitory action is useful as a therapeutic agent for a cartilage-related disease because it increases the amount of cartilage by chondrocyte differentiation promoting action. That is, it means that cartilage is regenerated by a substance having SIK2 inhibitory action.

Further, for example, in this example, by adding an effective amount of a low molecular weight compound having SIK2 inhibitory action in place of an antisense oligonucleotide against SIK2, chondrocyte differentiation promoting action of the low molecular weight compound having SIK2 inhibitory action can be confirmed.

### [Formulation Example]

### Formulation Example 1

By mixing the following components by a conventional method and then tableting, about 9000 tablets containing 10 mg of an active ingredient in one tablet can be obtained.

**[Table 2]**

| | |
|---|---|
| SIK2 ASO (SEQ ID NO: 1) | 100g |
| Carboxymethylcellulose calcium (disintegrant) | 20g |
| Magnesium stearate (lubricant) | 10g |
| Microcrystalline cellulose | 870g |

### INDUSTRIAL APPLICABILITY

A substance having SIK2 inhibitory action is useful as a therapeutic agent for a cartilage-related disease because it has chondrocyte differentiation promoting action.

### [Sequence Listing Free Text]

SEQ ID NO: 1: SIK2 antisense oligonucleotide
SEQ ID NO: 2: SIK2 antisense oligonucleotide
SEQ ID NO: 3: SIK2 antisense oligonucleotide
SEQ ID NO: 4: SIK2 antisense oligonucleotide
SEQ ID NO: 5: SIK2 antisense oligonucleotide
SEQ ID NO: 6: SIK3 antisense oligonucleotide

## Claims

1. A therapeutic agent for a cartilage-related disease, comprising a substance having SIK2 inhibitory action.

2. The therapeutic agent according to claim 1, wherein the cartilage-related disease is cartilage damage, articular disc damage, meniscus damage, osteoarthritis, chondrodysplasia, achondroplasia, achondrogenesis, dyschondrogenesis, chondrodystrophia, articular chondrocalcinosis, disk herniation, keypuncher's disease, temporomandibular joint disease, cartilage defect, anotia, microtia, osteochondrosis, spondylosis deformans, osteochondral defect, osteomalacia, rickets, osteoporosis, renal osteodystrophy, Paget's disease of bone, repair and repair failure of fracture, refracture, osteosarcoma or myeloma.

3. The therapeutic agent according to claim 1, wherein the cartilage-related disease is cartilage degeneration associated with a disease selected from rheumatoid arthritis, periarthritis scapulohumeralis, acute purulent arthritis, tuberculous arthritis, syphilitic arthritis, systemic lupus erythematosus, osteitis fibrosa, relapsing polychondritis, and osteochondritis dissecans.

4. The therapeutic agent according to any one of claims 1 to 3, for regenerating cartilage.

5. A medicine for treatment of a cartilage-related disease, comprising a substance having SIK2 inhibitory action in combination with at least one selected from a bone morphogenetic protein, an anti-inflammatory steroid, a non-steroidal anti-inflammatory drug, duloxetine, capsaicin, prostaglandin, a growth factor, a parathyroid hormone drug, a vitamin D derivative, a vitamin A derivative, a metalloproteinase inhibitor, a phosphodiesterase 4 inhibitor, an elastase inhibitor, an NFκB decoy oligo, a chondrocyte proliferation promoting factor, a chondroprotector, and an SIK3 inhibitor.

6. The therapeutic agent for a cartilage-related disease according to any one of claims 1 to 4, wherein the therapeutic agent is administered in combination with at least one selected from a bone morphogenetic protein, an anti-inflammatory steroid, a non-steroidal anti-inflammatory drug, duloxetine, capsaicin, prostaglandin, a growth factor, a parathyroid hormone drug, a vitamin D derivative, a vitamin A derivative, a metalloproteinase inhibitor, a phosphodiesterase 4 inhibitor, an elastase inhibitor, an NFκB decoy oligo, a chondrocyte proliferation promoting factor, a chondroprotector, and an SIK3 inhibitor.

7. A therapeutic agent for a cartilage-related disease comprising at least one selected from a bone morphogenetic protein, an anti-inflammatory steroid, a non-steroidal anti-inflammatory drug, duloxetine, capsaicin, prostaglandin, a growth factor, a parathyroid hormone drug, a vitamin D derivative, a vitamin A derivative, a metalloproteinase inhibitor, a phosphodiesterase 4 inhibitor, an elastase inhibitor, an NFκB decoy oligo, a chondrocyte proliferation promoting factor, a chondroprotector, and an SIK3 inhibitor, wherein the therapeutic agent is administered in combination with a substance having SIK2 inhibitory action.

8. A method for treating a cartilage-related disease, comprising administering an effective amount of a substance having SIK2 inhibitory action to a mammal.

9. A use of a substance having SIK2 inhibitory action for producing a therapeutic agent for a cartilage-related disease.

10. A substance having SIK2 inhibitory action to be used to treat a cartilage-related disease.

11. A method for screening a substance that promotes chondrocyte differentiation and/or has cartilage regenerating action, comprising:
(1) a step of measuring expression or function of SIK2 in presence of a test substance, and
(2) a step of selecting a test substance in which the expression or function of SIK2 is suppressed in step (1).

12. A chondrocyte differentiation promoter comprising a substance having SIK2 inhibitory action.

13. A cartilage regenerating agent comprising a substance having SIK2 inhibitory action.
